# DEMANDE DE BREVET EUROPEEN

(11) **EP 1 270 014 A1**
(43) Date de publication de la demande: **02.01.2003**
(21) Numéro de dépôt: 02291548.2
(22) Date de dépôt: 20.06.2002
(51) Int. Cl.: A61K 47/34

(54) **Composition semi-solide injectable biodégradable assurant un profil de libération particulier**

(30) Priorité: 20.06.2001 FR 0108129
(71) Demandeur: CEVA SANTE ANIMALE, 33500 Libourne (FR)
(72) Inventeur: Zanello, Philippe, 33130 Begles (FR); Boivin, Elianne, 33000 Bordeaux (FR); Kaltsatos, Vassilios, 33500 Libourne (FR); Capancioni, Sergio, 1880 Bex (CH); Schwach-Abdellaoui, Khadija, 74160 Collonges sous Saleve (FR); Gurny, Robert, 1204 Geneve (CH)
(74) Mandataire: Bernasconi, Jean Raymond

(57) **Abrégé**

La présente invention concerne une composition semi-solide injectable biodégradable assurant un profil de libération particulier caractérisé par la chute brutale de la libération de substance active après établissement d'un taux pratiquement constant de libération prolongée de ladite substance active, ladite composition comprenant :
- une substance active ;
- un polyorthoester semi-solide de formule (I). 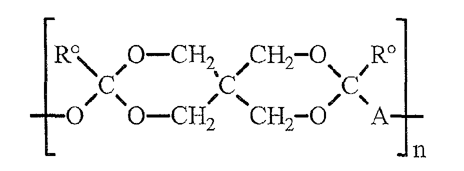
dans laquelle R°, A et n sont tels que définis à la revendication 1,
- éventuellement en association avec un ou plusieurs excipients miscibles audit polyorthoester.

## Description

L'invention concerne une composition semi-solide injectable biodégradable assurant un profil de libération prolongée particulier caractérisé par la chute brutale de la libération de substance active après établissement d'un taux pratiquement constant de libération de ladite substance active.

Plus précisément, les compositions de l'invention :
- assurent une libération rapide de la substance active de façon à atteindre rapidement des teneurs pharmacologiquement actives, sans toutefois atteindre des concentrations susceptibles d'induire des effets secondaires néfastes ;
- permettent l'établissement d'un taux de libération de la substance active pratiquement constant ; et
- assurent, en phase terminale de libération, une chute rapide des teneurs pharmacologiquement actives.

Chez l'animal, des compositions possédant une telle propriété présentent un intérêt majeur dans le domaine des traitements destinés à modifier les cycles sexuels des femelles, en particulier chez les espèces dites de rente (bovins, porcins, ovins, caprins, chevaux).

Chez ces espèces, l'insémination artificielle est fréquemment utilisée, de manière à obtenir une diffusion large des caractéristiques amélioratrices de la race ou de certains caractères recherchés.

Cependant dans les conditions d'élevage naturelles, l'insémination artificielle est parfois difficile à mettre en oeuvre pour des raisons pratiques. Lorsqu'un élevage est constitué d'un nombre important d'animaux à inséminer, on régule le cycle sexuel des femelles, pour synchroniser les périodes de venue en chaleurs. Toutes les femelles arrivent en chaleur en même temps, ce qui permet une utilisation plus économique de la semence et diminue aussi la période de manipulation des animaux. Parmi les techniques de régulation des cycles sexuels, une technique couramment utilisée consiste en l'administration, par différents systèmes, de composés à effet progestatif entraînant un blocage du cycle ovarien. Ces composés doivent être présents dans l'organisme pendant une période de temps déterminée puis doivent ensuite disparaître rapidement pour permettre le déclenchement d'un nouveau cycle sexuel.

Dans cet objectif, différents systèmes intravaginaux sont actuellement sur le marché. Par exemple, nous pouvons citer le PRID (Progesterone Releasing Intravaginal Device) et le CIDR (Controlled Internai Drug Release).

Le PRID est un système intravaginal, à base de progestérone. De forme spiralée, il contient 1,55 g de progestérone dissoute dans une matrice en silicone de 2 mm d'épaisseur. Le dispositif est placé dans le vagin de la femelle et la progestérone est libérée de façon régulière par diffusion à travers le réseau de silicone, (les taux plasmatiques atteignant 2 à 3 ng/ml). La progestérone bloque ainsi le cycle sexuel. Après une période de 10 à 14 jours, le produit est retiré de façon à ce que le cycle sexuel de l'animal reprenne. L'ovulation peut être déclenchée par injection de substances lutéolytiques telles que les prostaglandines.

Le CIDR est un système intravaginal, en forme de T, constitué d'un support en matière plastique, recouvert d'une fine couche de silicone. Le silicone est chargé en progestérone à hauteur 1,9 g par dispositif. L'utilisation du CIDR est identique à celle du PRID.

Chez les ovins, les systèmes intravaginaux ont été développés dans les années 70. Ces systèmes sont des éponges en polyuréthane (Syncro-part® des laboratoires Ceva Santé Animale et Chronogest® des laboratoires Intervet), dans lesquelles une hormone, l'acétate de fluorogestone (FGA) est dispersée sous forme pulvérulente. Au contact des fluides vaginaux, la FGA se dissout et passe ensuite dans la circulation sanguine. Les taux plasmatiques sont maintenus entre 1,5 et 3 ng/ml pendant toute la durée du traitement (10 à 14 jours). Lorsque les éponges sont retirées, les taux plasmatiques chutent brutalement pour revenir à une valeur proche de zéro sur une période de 12 à 24h. On peut citer aussi le Veramix® des laboratoires Pharmacia Santé Animale, qui est une éponge intravaginale contenant de l'acétate de médroxyprogestérone (MAP) ainsi que le CIDR destiné aux ovins.

Ces systèmes bien que largement utilisés depuis une trentaine d'année, présentent plusieurs inconvénients :
1. Il est nécessaire d'utiliser un applicateur spécial pour poser ces systèmes et la pose n'est pas toujours aisée.
2. La présence d'un système dans le vagin pendant une longue période d'une part gène les animaux et d'autre part provoque des phénomènes d'irritation aboutissant à l'apparition de muco-purulences plus ou moins importantes.
3. Ces systèmes ne sont pas très économiques puisqu'il a été montré qu'environ 50% du principe actif est encore présent dans les systèmes lorsqu'on les retire du vagin après la période souhaitée.
4. Il est nécessaire de retirer le système à l'issue de la période souhaitée pour provoquer la chute du taux sanguin d'hormones libérées par le système.
5. Il existe aussi un problème lié au recyclage de ces systèmes qui est rendu difficile par la présence de quantité importante d'hormones lorsqu'on jette le produit après utilisation.

Des systèmes injectables pourraient pallier aux inconvénients mentionnés ci-dessus, cependant, la demi-vie courte de ces hormones demanderait de nombreuses injections pour pouvoir maintenir les animaux en état d'anoestrus sur une période de 10 à 14 jours. Il existe cependant des implants destinés à être injectés par voie sous-cutanée. Ces implants sous forme de bâtonnets sont constitués d'une matrice silicone et contiennent du norgestomet. La pose de l'implant nécessite un appareil spécial et il faut aussi retirer l'implant de façon à ce que les taux sanguins de norgestomet retombent à zéro avant l'insémination. On constate aussi que pour ce type de système, une partie importante de l'hormone n'est pas consommée.

Des systèmes injectables biodégradables permettant la libération prolongée des substances actives ont par ailleurs été proposés dans la technique.

De nombreux brevets décrivent des systèmes injectables biodégradables sous forme de microparticules ou microcapsules, obtenues par différentes techniques. Par exemple, la demande WO 99/42110 décrit une méthode pour réguler l'oestrus de jeunes truies primipares. Cette méthode est basée sur l'induction de la puberté par injection d'une composition commerciale contenant une association de Pregnant Mare's Serum Gonadotrophin (PMSG) et Human Chorionic Gonadotropin (HCG), puis l'induction d'une pseudogestation par l'injection de 17β estradiol ou un analogue puis l'induction de l'oestrus par une injection d'une prostaglandine F2α ou un analogue. La composition contenant le 17β estradiol ou analogue est biodégradable, le polymère étant de préférence un copolymère des acides lactique et glycolique (PLAGA).

Cette composition se présente sous forme de microparticules, qui doivent être mises en suspension dans un véhicule avant d'être injectée. Le principe actif est libéré sur une période de 25 à 30 jours.

Souvent, ces microparticules sont difficiles à fabriquer à une échelle industrielle et par ailleurs, il est nécessaire de les disperser, le plus souvent dans l'eau, pour les injecter. Enfin, ces microparticules ou microcapsules ne permettent pas toujours de libérer le principe actif selon une cinétique d'ordre zéro.

Des alternatives à ces microparticules ont été proposées. Elles consistent en des préparations prêtes à l'emploi, le plus souvent constituées d'un polymère biodégradable dissout dans un solvant, ce qui permet d'obtenir un produit visqueux injectable (US 4 938 763 et 5 278 201). Une fois la composition injectée, le solvant diffuse dans l'organisme et le polymère forme un implant solide.

Un des inconvénients majeurs de ces systèmes est lié à ce que l'on appelle l'effet de jet (ou "burst effect").

Lorsque le solvant diffuse dans l'organisme, il entraîne avec lui une partie non négligeable du principe actif. Ainsi, le profil de libération de la substance active est caractérisé en ce cas par la libération immédiate d'une quantité relativement importante de principe actif, ce qui est particulièrement gênant lorsqu'on souhaite une libération prolongée du principe actif. La quantité initialement libérée peut atteindre de 30 à 75% en poids de la quantité totale à administrer.

Les demandes PCT WO 98/27962 et WO 98/27963 enseignent une façon de limiter l'effet de jet en ajoutant à la composition un émulsifiant qui peut être simplement de l'eau ou un alcool. Ces demandes divulguent des compositions sous forme de gels dans lesquelles le principe actif est associé à un polymère (copolymère des acides lactiques et glycoliques) dissout dans un solvant (triacétine ou N-méthylpyrrolidone). Ces compositions peuvent être complétées par l'ajout d'un "émulsifiant" qui permet d'une part de diminuer la viscosité du système injecté et d'autre part de limiter l'effet de jet. Si pour des raisons de stabilité le principe actif ne peut pas être incorporé dans ces compositions, le déposant propose un kit composé d'une part du système visqueux (solvant + polymère), d'autre part de l'émulsifiant et enfin du principe actif, le tout devant être mélangé juste avant emploi.

Ces systèmes diminuent l'effet de jet sans pour autant l'éliminer totalement, même après incorporation d'un excipient.

Par ailleurs, l'utilisation de solvants est nécessaire, ce qui peut poser des problèmes de tolérance locale lors de l'injection.

L'invention vise à résoudre ces différents problèmes en utilisant des polymères semi-solides pouvant être incorporés dans des compositions injectables à température ambiante sans l'utilisation de solvants assurant la solubilisation du polymère et/ou du principe actif. L'invention permet ainsi la préparation de compositions injectables à base de polyorthoester exemptes de l'un ou plusieurs des solvants conventionnellement utilisés tels que : la triacétine ; la N-méthyl-2-pyrrolidone ; la 2-pyrrolidone ; le glycérol formai ; l'acétate de méthyle ; le benzoate de benzyle ; l'acétate d'éthyle ; la (méthyl)(éthyl)cétone ; le diméthylformamide ; le diméthylsulfoxyde ; le tétrahydrofurane ; le caprolactame ; le décylméthylsulfoxyde ; l'acide oléique ; et la 1-dodécylazacycloheptanone.

Ce dernier point est particulièrement important dans le cas de principes actifs sensibles tels que les hormones, les peptides ou acides nucléiques. Les polymères semi-solides proposés dans la présente invention sont hydrophobes, subissent une dégradation en surface et libèrent la substance active de manière constante selon une cinétique contrôlée.

Après injection d'une telle composition, les taux plasmatiques de substance active sont rapidement atteints, un taux de relargage pratiquement constant assure ensuite la libération continue de substance active sur une durée modulable et enfin, les taux plasmatiques chutent brutalement jusqu'à des valeurs pour lesquelles le principe actif n'a plus de bénéfice thérapeutique.

Plus précisément, l'invention concerne une composition semi-solide injectable biodégradable assurant un profil de libération prolongé particulier caractérisé par la chute brutale de la libération de substance active après établissement d'un taux pratiquement constant de libération prolongée de ladite substance active, ladite composition comprenant :
- une substance active ;
- un polyorthoester semi-solide de formule (I) : 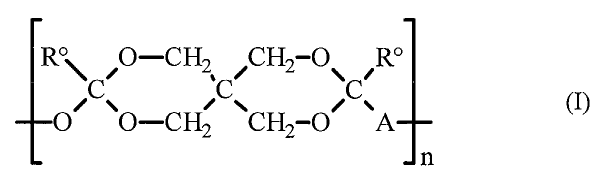 dans laquelle :
   R° représente C₁-C₄ alkyle ;
   n est un entier supérieur ou égal à 5 ;
   A représente un radical divalent choisi parmi -O-R¹- ; et -(O-R³)_{q}- où q est un entier de 1 à 20 ;
   R¹ a pour formule -(CHR⁴-CO-O)ₚ-R⁵- où
      - p est un entier de 1 à 20 ;
      - R⁴ est H ou (C₁-C₆) alkyle ; et
      - R⁵ représente
         -(CH₂-CH₂-O)ₛ-CH₂-CH₂- où s est un entier de 1 à 100 ; 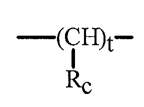
      où t est un entier de 1 à 30 et
      R_{c} est choisi parmi H et (C₁-C₆)alkyle ;
   et lorsque q représente 1 alors R³ est choisi parmi :
      -(CH₂-CH₂-O)ₓ-CH₂-CH₂- où x est un entier compris entre 1 et 100 ; 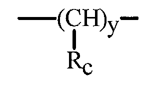 où y est un entier de 1 à 30 et
      R_{c} est choisi parmi H et (C₁-C₆)alkyle ;
      -R⁶-O-CR⁸R⁹-O-R⁷- où R⁶ et R⁷ sont indépendamment choisis parmi (C₁-C₁₂) alkylène ; R⁸ représente H ou (C₁-C₆) alkyle ; R⁹ représente (C₁-C₆) alkyle; ou bien R⁸ et R⁹ représentent ensemble (C₃-C₁₀) alkylène ;
   et lorsque q est un entier de 2 à 20, chaque R³ qui peut être identique ou différent est choisi parmi :
      -(CH₂CH₂-O)ₓ-CH₂CH₂- où x est tel que défini ci-dessus ;
      -(CH₂)_{y}- où y est tel que défini ci-dessus ; 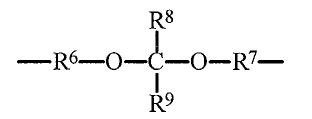 où R⁶, R⁷, R⁸ et R⁹ sont tels que définis ci-dessus ; ou 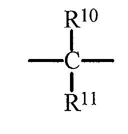 où R¹⁰ représente H ou (C₁-C₄) alkyle ;
      et R¹¹ représente (C₁-C₄) alkyle
   étant entendu que, pour au moins 0,1% en mole des motifs récurrents du polyorthoester, A représente -O-R¹- .
- éventuellement en association avec un ou plusieurs excipients miscibles audit polyorthoester.

Dans la formule (I) ci-dessus, la proportion de motifs récurrents contenant le radical divalent -O-R¹- (signification de A) détermine la vitesse d'hydrolyse du polyorthoester dans l'organisme et par conséquent la vitesse de dégradation du polymère.

Il doit être entendu que lorsque A représente -O-R¹- pour plus d'un motif récurrent, chaque R¹ peut être identique ou différent.

Lorsque A représente -(OR³)-_{q} pour plus d'un motif récurrent, chaque R³ peut être identique ou différent.

De façon générale, pour 0,1 à 100% en mole des motifs récurrents du polymère, A représente -OR¹-.

Dans le radical -O-R¹-, on préfère que p soit un entier compris entre 1 et 6, mieux encore entre 1 et 4, par exemple 1 ou 2 ; que R⁴ représente un atome d'hydrogène ou un groupe méthyle ; et que dans la définition de R⁵, s soit un entier compris entre 2 et 12, mieux encore entre 2 et 6, par exemple 2 ; t soit un entier compris entre 4 et 12, mieux encore entre 8 et 12, par exemple 10 ; et que R_{c} représente un atome d'hydrogène ou un groupe méthyle.

Dans le radical -(O-R³)_{q}-, on préfère que q représente un entier compris entre 1 et 6, mieux encore entre 1 et 3 ; et que dans les définitions de R³
- x est un entier de 2 à 12, mieux encore de 2 à 6, par exemple 2 ;
- y est un entier de 4 à 12, mieux encore de 4 à 6, par exemple 6 ;
- R⁶ et R⁷ sont identiques et représentent un groupe (C₄-C₁₂) alkylène linéaire, mieux encore un groupe (C₆-C₁₂) alkylène linéaire ;
- R⁸ est un atome d'hydrogène ;
- R⁹ est un groupe méthyle ;
- R¹⁰ est un atome d'hydrogène ; et
- R¹¹ est un groupe méthyle.

Selon un mode de réalisation particulièrement préféré, le polyorthoester est un polymère de formule (I) dans laquelle A représente -O-R¹- ou -O-R³-, dans lesquels R¹ représente -(CHR⁴-CO-O)ₚ-R⁵-
où R⁴ est tel que défini ci-dessus ;
p est un entier compris entre 1 et 20 ;
R⁵ et R³ sont indépendamment choisis parmi
-(CH₂-CH₂-O)ₐ-CH₂-CH₂- où a est un entier compris entre 1 et 30; et 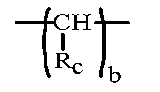 où b est un entier compris entre 2 et 30, et R_{c} est tel que défini ci-dessus ;
étant entendu que pour au moins 0,1% en mole des motifs récurrents du polyorthoester, A représente -O-R¹-.

Parmi ces polyorthoesters, on préfère que a représente 1 à 10 ; b représente 2 à 20, mieux encore 4 à 12 ; p représente 1 à 10, mieux encore 1 à 5 ; et R⁴ et R_{c} soient indépendamment choisis parmi un atome d'hydrogène et un groupe méthyle.

De façon générale, le polyorthoester est constitué de 5 à 100 motifs récurrents et présente une masse moléculaire comprise entre 1000 et 20000 Da, plus préférablement entre 2000 et 10000 Da.

De façon avantageuse, dans ledit polyorthoester, pour 5 à 80% en mole des motifs récurrents, A représente -O-R¹-.

De manière préférée, R⁴ représente -CH₃ dans la formule (I) du polyorthoester.

La nature des groupes en extrémité des polyorthoesters de formule I n'est pas critique selon l'invention et dépend du stoppeur de chaîne utilisé dans la synthèse dudit polyorthoester.

Le contrôle de la longueur de la chaîne polymérique, lequel est nécessaire pour l'obtention d'un polyorthoester semi-solide facilement injectable, est réalisé par addition, au milieu réactionnel, d'un stoppeur de chaîne (réactif arrêtant la propagation des chaînes polymériques). Des stoppeurs de chaîne appropriés sont des alcools monofonctionnels.

Des exemples d'alcools monofonctionnels sont les alcanols linéaires ou ramifiés en C₁-C₄₀, de préférence en C₁-C₂₀.

De préférence, la longueur de chaîne dudit stoppeur est la même que celle des diols utilisés pour la synthèse du polyorthoester qui ont respectivement pour formule :

HO-R¹-OH,

et

HO-(O-R³)_{q}-OH

où R¹, R³ et q sont tells que définis ci-dessus.

De manière avantageuse, on utilise comme stoppeur de chaîne les alcools monofonctionnels ayant pour formules :

H-R¹-OH,

HO-R¹-H,

H-(R³)_{q}-OH,

ou

HO-(R³)_{q}-H,

dans lesquels R¹, R³ et q sont tels que définis ci-dessus.

Le stoppeur de chaînes est utilisé lors de la polymérisation dans des proportions de 1 à 30 moles pour cent, de préférence 5 à 20 moles pour cent du nombre total de moles réactives. Les poly(orthoesters) ainsi préparés ont des faibles masses moléculaires et une polydispersité étroite et sont particulièrement souhaitables pour la présente invention.

Les compositions de l'invention comprennent préférablement de 50 à 99,9% en poids dudit polyorthoester, mieux encore de 80 à 99,9% en poids.

Les compositions de l'invention sont plus particulièrement appropriées, du fait de la cinétique de libération particulière du principe actif à l'administration chez l'animal d'hormones ou analogues capables de bloquer le cycle sexuel pendant une période de temps équivalente à la durée de présence d'un corps jaune ovarien.

Les compositions de l'invention miment en effet la pharmacocinétique d'un système qui serait posé puis retiré 10 à 15 jours après, tel qu'un système intravaginal ou un implant non dégradable.

Après cette période, l'injection d'une autre hormone puis l'insémination sont alors possibles.

La période de fin d'administration coïncide en fait avec l'érosion complète du polyorthoester et sa disparition complète de l'organisme.

Des exemples de principes actifs capables de bloquer le cycle sexuel chez l'animal sont notamment la fluorogestone, la progestérone, la médroxyprogestérone, l'altrénogest, le norgestomet, le mégestrol, la chlormadinone, les analogues de l'hormone de libération des gonadotrophines (GnRH), la mélatonine et leurs sels pharmaceutiquement injectables.

Plus préférablement, on utilise à titre de substance active la fluorogestone ou l'un de ses sels pharmaceutiquement acceptables, tel que l'acétate de fluorogestone.

Lorsque la substance active est une hormone endogène telle que la progestérone, celle-ci sera préférablement contenue dans la composition à raison de 5 à 50% en poids du poids total de la composition, mieux encore à raison de 5 à 30% en poids.

Lorsque la substance active est une hormone de synthèse ou un analogue, celle-ci sera préférablement contenue dans la composition à raison de 0,1 à 10% en poids, mieux encore à raison de 0,1 à 5,0% en poids. Des exemples de telles substances actives sont la fluorogestone et ses sels pharmaceutiquement acceptables ou le norgestomet.

De façon à moduler la cinétique de libération, il est par ailleurs possible d'incorporer au mélange de la substance active et du polyorthoester, un excipient parmi ceux classiquement utilisés dans la préparation des compositions injectables.

Un tel excipient est par exemple une huile, un polyéthylèneglycol, une substance tampon telle qu'un tampon phosphate, sulfate, acétate, citrate, succinate, maléate, un ester d'acide gras, un acide carboxylique ou un de leurs mélanges.

Des huiles particulièrement avantageuses sont les huiles végétales telles que l'huile de palmiste, l'huile de palme, l'huile de son de riz, l'huile d'arachide, l'huile de colza, l'huile de babassu, l'huile de coprah, l'huile d'ouricury, l'huile de tournesol, l'huile de sésame ou l'huile d'olive, ainsi que leurs dérivés plus ou moins hydrogénés. De manière préférée, on utilisera l'huile de sésame ou l'huile d'olive.

Les esters d'acide gras sont notamment les esters dérivés d'acide gras à chaîne linéaire ou ramifiée présentant jusqu'à 24 atomes de carbone.

Comme acide gras à chaîne ramifiée, on peut mentionner l'acide 2-éthylhexanoïque, l'acide isopélargonique, l'acide isomyristique et l'acide isostéarique.

Comme acide gras à chaîne droite, on peut mentionner l'acide valérique, l'acide caproïque, l'acide énanthoïque, l'acide caprylique, l'acide pélargonique, l'acide caprique, l'acide laurique, l'acide myristique, l'acide arachique, l'acide béhénique, l'acide lignocérique, l'acide palmitoléique, l'acide oléique, l'acide élaïdique, l'acide érucique, l'acide sorbique et l'acide linoléique.

Des exemples d'esters d'acide gras sont les esters formés avec des alcools inférieurs à chaîne hydrocarbonée linéaire ou ramifiée présentant de 1 à 10 atomes de carbone, de préférence de 1 à 6 atomes de carbone, par exemple de 1 à 4 atomes de carbone, tels que le méthanol, l'éthanol et le propanol. On utilisera plus particulièrement l'oléate d'éthyle.

A titre d'ester d'acide gras particulièrement préféré, on peut citer l'oléate d'éthyle.

D'autres esters d'acide gras sont par exemple les mono-, di- ou triglycérides d'acide gras.

De façon avantageuse, l'acide gras de ces glycérides présente jusqu'à 18 atomes de carbone.

Des exemples en sont plus particulièrement le caprylate de glycéryle ; le caprate de glycéryle ; le caprate / caprylate de glycéryle ; le triglycéride des acides capriques et capryliques ; et le triglycéride des acides caprylique, caprique et laurique.

D'autres esters d'acide gras sont les esters formés à partir du propylène glycol.

A titre d'excipient, on peut utiliser également un polyéthylèneglycol ou un dérivé mono- ou diétheré de celui-ci.

Les polyéthylèneglycols utilisables ont généralement une masse moléculaire comprise entre 400 et 3000, de préférence entre 500 et 1500.

Les acides carboxyliques utilisables sont les acides mono- ou polycarboxyliques à chaîne linéaire ou ramifiée présentant de 1 à 24 atomes de carbone. Ils comprennent notamment les acides gras définis ci-dessus. Plus généralement, on pourra incorporer à la composition de l'invention un acide organique quelconque.

Généralement, les excipients seront présents dans la composition à raison de 5 à 40% en poids du poids total de la composition.

La modulation de la durée de libération in vivo (profil pharmacocinétique) est réalisée en jouant sur certains facteurs comme le pourcentage d'incorporation du principe actif, la nature et la teneur de chacun des excipients utilisés.

La composition peut contenir d'autres additifs usuels tels que les agents stabilisants, les agents conservateurs antimicrobiens et antioxydants habituellement utilisés dans les produits pharmaceutiques injectables.

On peut éventuellement envisager d'inclure à ces compositions des vitamines, agents nutritifs ou médicaments.

Les compositions de l'invention peuvent être préparées par mise en oeuvre d'un procédé comprenant une première étape de mélange de la substance active audit polyorthoester de formule (I), et éventuellement une deuxième étape comprenant l'incorporation d'excipients et/ou d'additifs à ce mélange. La substance active, les excipients et/ou les additifs sont sous forme solvatée ou sous forme dispersée dans la matrice polymère.

Selon un mode de réalisation particulièrement avantageux, la substance active, de même que les excipients et/ou additifs, sont incorporés au mélange réactionnel lors de la synthèse dudit polyorthoester.

Les compositions semi-solides de l'invention sont particulièrement avantageuses dans la mesure où :
- elles sont prêtes à l'emploi et directement injectables ;
- elles sont préparées par simple mélange des ingrédients ou encore plus avantageusement, en une seule étape, lors de la synthèse du polyorthoester ;
- elles ne comportent pas de solvant, en particulier aucun solvant susceptible d'exercer des effets irritants au site d'injection.

Selon un autre de ses aspects, l'invention concerne l'utilisation d'un polyorthoester de formule (I) tel que défini ci-dessus en association avec une substance active pour la préparation d'une composition injectable biodégradable en vue de l'administration de ladite substance active à l'homme ou à l'animal selon un profil de libération prolongée particulier caractérisé par la chute brutale de la libération prolongée de substance active après établissement d'un taux pratiquement constant de libération de ladite substance active.

Les compositions de l'invention sont injectables chez les mammifères et notamment les bovins, ovins, porcins, caprins, chevaux, chats et chiens, par voie sous-cutanée ou intramusculaire.

L'injection est réalisable avec un injecteur muni d'une aiguille dont le diamètre est ajusté de façon à ce que la composition puisse être injectée sans qu'il soit nécessaire d'exercer une pression très forte. Un exemple d'injecteur est l'injecteur de type Ligmaject® commercialisé par la Société Kaladent S.A.utilisé pour les injections de produits semi-solides dans le domaine dentaire.

L'invention est illustrée ci-dessous à la lumière des exemples suivants.

Dans tous ces exemples, le polyorthoester (1) est un polymère de formule I dans laquelle :
R° est CH₂-CH₃
A est -O-R¹- pour 30% en moles de motifs récurrents, pour le reste des motifs récurrents A étant -(-O-R³-)_{q}- avec :

   R¹ = -(-CHR⁴-CO-O-)ₚ-R⁵

   p étant compris entre 1 et 5
   R⁴ = CH₃ 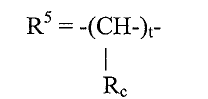 R_{c} = H
   t = 10
   q = 1
R³ est ―(-CH₂-)_{y}- où y = 10.

Par ailleurs, 15% en moles des motifs récurrents pour lesquels A représente -(O-R₃-)-_{q} sont remplacés par l'unité monovalente -O-(CH₂-)ₙ-CH₃ où n = 9, ces groupements étant respectivement situés aux deux extrémités de la chaîne polymérique.

### EXEMPLE 1

Par simple mélange à température ambiante des différents constituants la composant, on prépare la composition suivante :

| Constituants | Pourcentage massique |
|---|---|
| Acétate de fluorogestone | 1,5% |
| Huile de sésame | 5,0% |
| Polyorthoester (1) | 93,5% |

### EXEMPLE 2

Par mise en oeuvre d'un protocole opératoire identique à celui de l'exemple 1, on prépare la composition suivante :

| Constituants | Pourcentage massique |
|---|---|
| Progestérone | 30% |
| Polyéthylèneglycol 1450 | 10% |
| Polyorthoester (1) | 60% |

### EXEMPLE 3

Par mise en oeuvre d'un protocole opératoire identique à celui de l'exemple 1, on prépare la composition suivante :

| Constituants | Pourcentage massique |
|---|---|
| Norgestomet | 0,3% |
| Acide citrique | 1,0% |
| Polyorthoester (1) | 98,7% |

### EXEMPLE 4

Cet exemple propose une étude du profil pharmacocinétique de libération in vivo du principe actif dans le cas de la composition de l'exemple 1.

Après injection sous-cutanée de la composition de l'exemple 1 chez la brebis, on observe un profil phamacocinétique avec des teneurs en FGA comprises entre 1 et 5 ng/ml, soit les taux plasmatiques observés lorsqu'on utilise une éponge par voie intravaginale (type syncro-part® ou chronogest®).

Après environ 12 jours, les taux plasmatiques chutent brutalement, cette chute plasmatique est proche de celle observée lorsqu'on retire une éponge du vagin d'un animal.

### EXEMPLE 5

La cinétique de libération *in vitro* de l'acétate de fluorogestone est étudiée en fonction de la nature et de la concentration en excipient et en fonction de la teneur en polyorthoester (1).

Le protocole opératoire consiste à placer le mélange substance active + polyorthoester (1) + excipient dans une cellule à flux continu. Cette cellule est traversée par un flux d'une solution tampon phosphate pH = 7,4. A la sortie de la cellule, le liquide d'extraction est prélevé puis analysé par chromatographie liquide haute pression pour déterminer la teneur en fluorogestone.

Les résultats obtenus sont rapportés dans le tableau suivant :

| Formulation de la composition testée | | | Pourcentage d'acétate de fluorogestone libéré in vitro après 14 jours |
|---|---|---|---|
| Acétate de fluorogestone (% en poids) | Excipient (% en poids) | Polyorthoester (1) (% en poids) | |
| 1,5 | 5,0 a | 93,5 | 17,4% |
| 3,0 | 5,0 a | 92 | 8,3% |
| 1,5 | 20,0 a | 78,5 | 10,1% |
| 1,5 | 2,5 b | 96 | 14,3% |
| 1,5 | 5,0 b | 93,5 | 20,2% |

| | | | |
|---|---|---|---|
| a : huile de sésame | | | |
| b : polyéthylèneglycol 1450 | | | |

Cet exemple montre bien qu'en variant la nature et les quantités respectives d'excipient et de polyorthoester, on peut modifier la cinétique globale de libération du principe actif.

## Revendications

1. Composition semi-solide injectable biodégradable assurant un profil de libération prolongée particulier **caractérisé par** la chute brutale de la libération de substance active après établissement d'un taux pratiquement constant de libération prolongée de ladite substance active, ladite composition comprenant :
• une substance active ;
• un polyorthoester semi-solide de formule (I) : 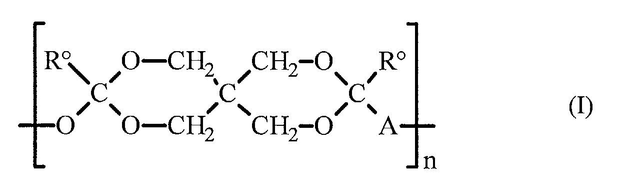 dans laquelle :
R° représente C₁-C₄ alkyle ;
n est un entier supérieur ou égal à 5 ;
A représente un radical divalent choisi parmi -O-R¹- ; et -(O-R³)_{q}- où q est un entier de 1 à 20 ;
R¹ a pour formule -(CHR⁴-CO-O)ₚ-R⁵- où
- p est un entier de 1 à 20 ;
- R⁴ est H ou (C₁-C₆) alkyle ; et
- R⁵ représente
-(CH₂-CH₂-O)ₛ-CH₂-CH₂- où s est un entier de 1 à 100 ; 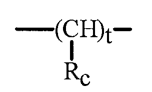 où t est un entier de 1 à 30 et
R_{c} est choisi parmi H et (C₁-C₆)alkyle ;
et lorsque q représente 1 alors R³ est choisi parmi :
-(CH₂-CH₂-O)ₓ-CH₂-CH₂- où x est un entier compris entre 1 et 100 ; 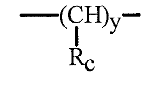 où y est un entier de 1 à 30 et
R_{c} est choisi parmi H et (C₁-C₆)alkyle ;
-R⁶-O-CR⁸R⁹-O-R⁷- où R⁶ et R⁷ sont indépendamment choisis parmi (C₁-C₁₂) alkylène ; R⁸ représente H ou (C₁-C₆) alkyle ; R⁹ représente (C₁-C₆) alkyle; ou bien R⁸ et R⁹ représentent ensemble (C₃-C₁₀) alkylène ;
et lorsque q est un entier de 2 à 20, chaque R³ qui peut être identique ou différent est choisi parmi :
- (CH₂CH₂-O)ₓ-CH₂CH₂- où x est tel que défini ci-dessus ;
- (CH₂)_{y}- où y est tel que défini ci-dessus ; 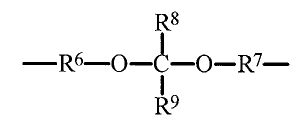 où R⁶, R⁷, R⁸ et R⁹ sont tels que définis ci-dessus ; ou 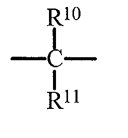 où R¹⁰ représente H ou (C₁-C₄) alkyle ;
et R¹¹ représente (C₁-C₄) alkyle
étant entendu que, pour au moins 0,1% en mole des motifs récurrents du polyorthoester, A représente -O-R¹- .
• éventuellement en association avec un ou plusieurs excipients miscibles audit polyorthoester.

2. Composition selon la revendication 1, **caractérisée en ce que** ledit polyorthoester de formule (I) est tel que :
A représente -O-R¹- ou -O-R³- dans lesquels R¹ représente -(CHR⁴-CO-O)ₚ-R⁵-
où R⁴ est tel que défini à la revendication 1 ;
p est un entier compris entre 1 et 20 ;
R⁵ et R³ sont indépendamment choisis parmi :
-(CH₂-CH₂-O)ₐ-CH₂-CH₂- où a est un entier compris entre 1 et 30; et 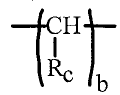 où b est un entier compris entre 2 et 30, et R_{C} est tel que défini ci-dessus ;
étant entendu que pour au moins 0,1% en mole des motifs récurrents du polyorthoester, A représente -O-R¹-

3. Composition selon la revendication 2, **caractérisée en ce que** dans ledit polyorthoester, pour 5 à 80% en mole des motifs récurrents, A représente -O-R¹-.

4. Composition selon l'une quelconque des revendications 1 à 3, **caractérisée en ce que** la masse moléculaire du polyorthoester varie entre 2000 et 10000 Da.

5. Composition selon l'une quelconque des revendications 1 à 4, **caractérisée en ce que** dans ledit polyorthoester, R⁴ représente CH₃.

6. Composition selon l'une quelconque des revendications 1 à 5, **caractérisée en ce qu'**elle renferme de 50 à 99,9% en poids dudit polyorthoester, mieux encore de 80 à 99,9% en poids.

7. Composition selon l'une quelconque des revendications précédentes, **caractérisée en ce que** le principe actif est choisi parmi la fluorogestone, la progestérone, la médroxyprogestérone, l'altrénogest, le norgestomet, le mégestrol, la chlormadinone, les analogues de l'hormone de libération des gonadotrophines (GnRH), la mélatonine et leurs sels pharmaceutiquement injectables.

8. Composition selon la revendication 7, **caractérisée en ce que** le principe actif est la fluorogestone ou l'un de ses sels pharmaceutiquement acceptables tel que l'acétate de fluorogestone.

9. Composition selon l'une quelconque des revendications 1 à 7, **caractérisée en ce qu'**elle contient de 5 à 50% en poids d'une hormone endogène telle que la progestérone, de préférence de 5 à 30% en poids.

10. Composition selon l'une quelconque des revendications 1 à 7, **caractérisée en ce qu'**elle contient de 0,1 à 10% en poids d'une hormone de synthèse ou d'un analogue de synthèse tel que la fluorogestone ou le norgestomet, de préférence de 0,1 à 5,0% en poids.

11. Composition selon l'une quelconque des revendications 1 à 10, **caractérisée en ce que** l'excipient miscible audit polyorthoester est une huile, un polyéthylèneglycol, une substance tampon, un ester d'acide gras, un acide carboxylique ou un de leurs mélanges.

12. Composition selon la revendication 11, **caractérisée en ce que** l'huile est une huile végétale telle que l'huile de sésame ou l'huile d'olive.

13. Composition selon la revendication 11, **caractérisée en ce que** l'ester d'acide gras est un mono-, di- ou triglycéride d'acide gras, l'acide gras présentant jusqu'à 18 atomes de carbone, ou bien l'oléate d'éthyle.

14. Composition selon l'une quelconque des revendications 11 à 13, **caractérisée en ce qu'**elle contient de 5 à 40% en poids d'excipient.

15. Utilisation d'un polyorthoester de formule (I) tel que défini à l'une quelconque des revendications 1 à 5 en association avec une substance active pour la préparation d'une composition injectable biodégradable en vue de l'administration de ladite substance active à l'homme ou à l'animal selon un profil de libération prolongée particulier **caractérisé par** la chute brutale de la libération de substance active après établissement d'un taux pratiquement constant de libération de ladite substance active.

16. Utilisation selon la revendication 15, **caractérisée en ce que** la composition injectable est destinée à bloquer temporairement le cycle sexuel d'un mammifère, la substance active étant une hormone ou un analogue capable de bloquer le cycle sexuel tel que défini à l'une quelconque des revendications 8 ou 9.

17. Procédé pour la préparation d'une composition semi-solide injectable biodégradable selon l'une quelconque des revendications 1 à 15, **caractérisé en ce que** l'on mélange dans une première étape la substance active audit polyorthoester de formule (I), sous forme dispersée ou solvatée et **en ce que** l'on incorpore éventuellement à ce mélange un excipient sous forme solvatée ou dispersée.

18. Procédé pour la préparation d'une composition semi-solide injectable biodégradable selon l'une quelconque des revendications 1 à 14, **caractérisé en ce que** ladite substance active et éventuellement ledit excipient sont ajoutés au mélange réactionnel lors de la synthèse dudit polyorthoester.
